(19) Europäisches Patentamt / European Patent Office / Office européen des brevets

(11) **EP 2 996 703 B1**

(12) **EUROPEAN PATENT SPECIFICATION**


(45) Date of publication and mention of the grant of the patent:
**07.03.2018 Bulletin 2018/10**

(21) Application number: **14732396.8**

(22) Date of filing: **16.05.2014**

(51) Int Cl.:
***A61K 36/482*** *(2006.01)* ***A61P 17/02*** *(2006.01)*

(86) International application number:
**PCT/IB2014/061488**

(87) International publication number:
**WO 2014/184779 (20.11.2014 Gazette 2014/47)**


(54) **SENNA EXTRACTS AND USES THEREOF**

SENNAEXTRAKTE UND VERWENDUNGEN DAVON

EXTRAITS DE SÉNÉ, ET UTILISATIONS DE CEUX-CI


(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.05.2013 IT RM20130294**

(43) Date of publication of application:
**23.03.2016 Bulletin 2016/12**



(73) Proprietor: **Aboca S.p.A. Società Agricola**
**52037 Sansepolcro (AR) (IT)**

(72) Inventor: **MERCATI, Valentino**
**I-52037 Sansepolcro (AR) (IT)**

(74) Representative: **Predazzi, Valentina et al**
**Società Italiana Brevetti S.p.A.**
**Piazza di Pietra, 39**
**00186 Roma (IT)**



(56) References cited:
**DE-A1- 4 120 991 GB-A- 2 018 588**

- **PENTTI HIETALA ET AL: "Laxative Potency and Acute Toxicity of Some Anthraquinone Derivatives, Senna Extracts and Fractions of Senna Extracts", PHARMACOLOGY AND TOXICOLOGY, vol. 61, no. 2, 1 August 1987 (1987-08-01) , pages 153-156, XP055128793, ISSN: 0901-9928, DOI: 10.1111/j.1600-0773.1987.tb01794.x**
- **Srivastava A, Pandey R, Verma RK, Gupta MM.: "Liquid chromatographic determination of sennosides in Cassia angustifolia leaves.", Pubmed J AOAC Int, vol. 89, no. 4 July 2006 (2006-07), XP002701177, Retrieved from the Internet: URL:http://www.ncbi.nlm.nih.gov/pubmed/16915828 [retrieved on 2013-07-10]**
- **DATABASE BIOSIS [Online] BIOSCIENCES INFORMATION SERVICE, PHILADELPHIA, PA, US; April 2006 (2006-04), SRIVASTAVA MANJOOSHA ET AL: "Pharmacognostical evaluation of Cassia angustifolia seeds", XP002701175, Database accession no. PREV200600493089 & SRIVASTAVA MANJOOSHA ET AL: "Pharmacognostical evaluation of Cassia angustifolia seeds", PHARMACEUTICAL BIOLOGY, vol. 44, no. 3, April 2006 (2006-04), pages 202-207, ISSN: 1388-0209**
- **DATABASE BIOSIS [Online] BIOSCIENCES INFORMATION SERVICE, PHILADELPHIA, PA, US; September 2004 (2004-09), SRIVASTAVA MANJOOSHA ET AL: "Pharmacognostic evaluation of Sanaai Seed - Cassia angustifolia", XP002701176, Database accession no. PREV200510047631 & SRIVASTAVA MANJOOSHA ET AL: "Pharmacognostic evaluation of Sanaai Seed - Cassia angustifolia", JOURNAL OF MEDICINAL AND AROMATIC PLANT SCIENCES, vol. 26, no. 3, September 2004 (2004-09), pages 543-547, ISSN: 0253-7125**



Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- CUELLAR M J ET AL: "Topical anti-inflammatory activity of some Asian medicinal plants used in dermatological disorders", MEDICINAL & AROMATIC PLANTS ABSTRACTS, vol. 23, no. 6, 1 December 2001 (2001-12-01), XP018012310, SCIENTIFIC PUBLISHERS, SCIENTIFIC PUBLISHERS, NEW DELHI - INDIA ISSN: 0250-4367
- WALTENBERGER B ET AL: "Transport of sennosides and sennidines from Cassia angustifolia and Cassia senna across Caco-2 monolayers - an in vitro model for intestinal absorption", PHYTOMEDICINE, vol. 15, no. 5, 15 May 2008 (2008-05-15), pages 373-377, XP022609720, GUSTAV FISCHER VERLAG, STUTTGART, DE ISSN: 0944-7113, DOI: 10.1016/J.PHYMED.2007.03.008 [retrieved on 2008-04-14]
- ANTON R ET AL: "THERAPEUTIC USE OF NATURAL ANTHRAQUINONE FOR OTHER THAN LAXATIVE ACTIONS", PHARMACOLOGY, vol. 20, no. SUPPL. 01, 1 January 1980 (1980-01-01), pages 104-112, XP001036900, S. KARGER AG, CH ISSN: 0031-7012, DOI: 10.1159/000137404
- DATABASE TKDL [Online] 1999, "Nadivrane Avallikadi Taila", XP002727222, Database accession no. RG2/838 & Anonymous: "Sodhala", 1999, Gadanigrahah, Varanasi vol. 3rd Edit, page 329, the whole document

## Description

The present invention is defined in claims 1-17. The present invention relates to *Cassia acutifolia* and/or *Cassia angustifolia* extracts depleted in sennosides as mucosal membrane, gastric- and/or dermoprotective agents, processes for the preparation thereof and compositions comprising such extracts.

STATE OF THE PRIOR ART

Senna (*Cassia acutifolia* or *Cassia angustifolia*) is a plant whose leaves or whose pods (follicles) are commonly used as laxatives.
The laxative action of senna is notoriously a markedly irritating laxative action, due to the presence, in pods and leaves, of anthraquinones known as sennosides.
The main active constituents in the leaf are sennosides A and B (plus the sennosides A1, C, D, G) about the 2.5% thereof expressed in content of sennoside B ($C_{42}H_{38}O_{20}$; Mr863 according to the European Pharmacopoeia), and in the pod, commonly also referred to as follicle, said components are about the 4%, expressed as content of sennoside B.
Sennosides action is due to their conversion, in the large intestine, into the active metabolite (rheinanthrone) which has an effect on large intestine motility (it stimulates peristaltic contractions and inhibits local contractions) with consequent acceleration of in-colon transit and reduction of liquid absorption and an effect on secretion effects (stimulation of mucus secretion and active chloride secretion) w an increase in liquid secretion.
The standard method for titration of sennosides in Senna leaves and pods is described in European Pharmacopoeia 7.0, in chapters titled Sennae folium (pages 1236 and 1237, European Pharmacopoeia 7.0 ed. 2011, 01/2008:0206 corrected 6.0). In the present description, the indication of total sennosides present in the extracts, indicated as "SFM total sennosides expressed as sennoside B" is that obtained by the above-indicated method, i.e. sennosides determined by spectrophotometric method according to what described in the abovecited European Pharmacopoeia.
Different quantification forms will be differently indicated.
Therefore, the use of senna leaves and/or follicles as laxative, and of their extracts as laxatives is known in the art, and also the irritating effect of such product on the intestine is known, both linked to sennosides presence in leaves as well as in pods of the senna plant.

SUMMARY OF THE INVENTION

The present invention relates to a senna extract substantially depleted in the known pharmacologically active components represented by total sennosides, of which only traces remain, and optionally depleted also in resins, and to a process for obtaining such extract. The extract according to the invention may be used for a purpose completely different from that for which the senna extract is used in the state of the art, i.e. as protective agent of the mucous membranes or of the skin. Such use can be carried out thanks to the protective activities of the remaining components, in which the extract is enriched by 15-25% with respect to the initial product thanks to the removal of total sennosides therefrom. The extract of the invention therefore provides a new source of material of plant origin useful for the protection of the mucous membranes and of the skin, wherein the pharmacological effects attributable to the components known as active principles are substantially reduced or eliminated.
Said condition is particularly desirable when materials of natural or plant origin are to be used for actions of a mainly mechanical type (i.e., not for pharmacological actions having, e.g., a direct effect on the activation of receptorial pathways, immune pathways or metabolic processes). The use of substances with a mainly mechanical effect, such as for instance substances having a barrier or mucoadhesive effect, is desirable, e.g., for assisting a pharmacological therapy. By minimizing the presence of substances with pharmacological activities in the material of natural or plant origin as in the present invention, a material with a mainly mechanical effect is obtained, which can be used in combination with drugs, minimizing possible undesired interactions with drugs and, at the same time, exploiting the typical feature of substances of plant origin, of having a vast number of components able to exert a protective effect besides the pharmacologically active ones commonly used.
The use of extracts of plant or natural complex substances depleted in pharmacologically active principles entails the dual advantage of providing a material having a mainly mechanical protective action (mucous membrane protection or skin protection) and of enabling a concrete and useful use of materials normally discarded in extraction processes of plant or natural active principles.
The present invention therefore relates to a senna extract depleted in total sennosides, wherein said total sennosides have a concentration in weight percentage of at least ≤ 0.5%; its use as protective agent of partially compromised skin or mucous membranes; compositions comprising such extract; their use in the protection of the skin or mucous membranes and processes for the preparation of such extract and of such compositions.
The invention also relates to a process for the preparation of Senna extract as defined above, comprising the

following steps:

a. preparing a hydroalcoholic extract of Senna leaves and/or pods (follicles) wherein said hydroalcoholic extract is prepared by subjecting *Cassia acutifolia* and/or *Cassia angustifolia* leaves and/or pods, optionally fragmented, to two or more extraction steps in ethanol at decreasing concentration
b. obtaining from the extract prepared in a. a concentrated aqueous extract and a resinous extract free from sennosides
c. concentrating said alcoholic extract by decantation and/or centrifugation and/or filtration, collecting the supernatant or the filtrate
d. said supernatant or filtrate in c. is subjected to one or more ultrafiltration steps whereby the permeate is collected and said permeate comprises total sennosides at a concentration in weight percentage ≤50.5% and wherein said one or more ultrafiltration steps is carried out on a semi-permeable membrane with a cutoff of about 500-15,000 daltons and/or

said filtrate or supernatant obtained in step c. or said permeate obtained after ultrafiltration is subjected to one or more steps through a high porosity adsorbing resin and the eluate is collected
wherein said eluate thus obtained comprises total sennosides at a concentration in weight percentage ≤0.5.

DETAILED DESCRIPTION OF THE FIGURES

Figure 1 shows a block diagram of an embodiment of the process of the invention.
Figure 2a shows a block diagram of step d and Figure 2b shows a block diagram of step d'.
Figure 3 shows a graph of mucoadhesion on an inclined plane (assay described below) in which the assayed substance is 50° ethanol in panel A (extract solvent), sodium alginate in panel B, the extract of the invention in panel C, water in panel D (sodium alginate solvent).

Squares denote results obtained on an inclined plane with mucin and the sample under examination, whereas rhombs denote those obtained on the control (blank) in which the sample under examination is assayed on an inclined plane without mucin.
Figure 3A shows that for 50% EtOH, a non-mucoadhesive substance, the measurements of the sliding on the inclined plane with and without mucin are practically overlappable.
Squares denote results obtained on an inclined plane with mucin and 50% EtOH, whereas rhombs denote those obtained on the control (blank) in which the sample under examination is assayed on an inclined plane without mucin.
Figure 3B shows a graph of mucoadhesion on an inclined plane (assay described below) in which the assayed substance is sodium alginate, a substance with known mucoadhesive properties.
Squares denote results obtained on an inclined plane with mucin and sodium alginate, whereas rhombs denote those obtained on the control (blank) in which sodium alginate is assayed on an inclined plane without mucin.
In the case of the sample consisting of sodium alginate; a remarkable difference in behavior is witnessed in the presence and in the absence of mucin.
Figure 3C shows a graph of mucoadhesion on an inclined plane (assay described below) in which the assayed substance is the senna extract depleted in resins and sennosides (obtainable, therefore, at point d. or d' of the process) according to the present description in order to verify its mucoadhesive properties.
Squares denote results obtained on an inclined plane with mucin and senna extract according to the present description, whereas rhombs denote those obtained on the control (blank) in which the senna extract according to the present description is assayed on an inclined plane without mucin.
Figure 3D shows a graph of mucoadhesion on an inclined plane (assay described below) in which the assayed substance is water.
Squares denote results obtained on an inclined plane with mucin and water whereas rhombs denote those obtained on the control (blank) in which water is assayed on an inclined plane without mucin.
The figure shows that for water, a non-mucoadhesive substance, the measurements of the sliding on the inclined plane with and without mucin are practically overlappable.
It may be noted how the extract has a behavior similar to sodium alginate; a remarkable difference in behavior is witnessed in the presence and in the absence of mucin, thereby resulting with mucoadhesive features.

SHORT GLOSSARY

NMWCO=Nominal Molecular weight Cut-off
FILTRATE: extract that has passed through the semi-permeable ultrafiltration membrane or through the panel filter
PERMEATE: extract that has passed through the ultrafiltration membrane.
RETENTATE: extract that did not pass through the ultrafiltration membrane or material adsorbed on resin
ELUATE: material not adsorbed on resin following adsorption passages thereon.
ULTRAFILTRATION: filtration technique on semi-permeable membrane characterized by pores having sizes of 100,000 daltons (about 0.1 μm) to 500 daltons (about 0.005μm)
By "NOT GREATER THAN", relative to the amounts of active principles in the extract, in the present description it is meant ≤.

By "total sennosides", in the present invention total SFM sennosides are meant, expressed as sennoside B as per reference in the description of the state of the art (European Pharmacopoeia 7.0 ed. 2011).
For the purposes of the present invention, the weight percentages reported refer to the dried extracts and are therefore weight percentages of the dried extracts.
For the purposes of the present invention, the term "senna" is used to denote *Cassia acutifolia* and/or *Cassia angustifolia.*

DETAILED DESCRIPTION OF THE INVENTION

As indicated above, in the present description a senna extract depleted in total SFM sennosides expressed as sennoside B is provided, wherein said total sennosides have a concentration in weight percentage of about ≤ 0.5%. By applying the extraction process object of the invention, described below, and separation techniques on semi-permeable membrane or on resin, it was possible to obtain a resinous senna extract free from sennosides and a senna extract depleted in resins and in total sennosides, and concomitantly surprisingly enriched in the remaining substances. Both extracts showed an effective protective action on the skin and on the mucous membranes, as evident from the mucoadhesivity assay and barrier assay reported in the experimental section below.
Both extracts are useful as described in the present invention and can also be mixed together so as to obtain a senna extract comprising resins and depleted in total sennosides.
Compared to the dry extracts of senna and to senna leaves and/or pods as such, which comprise an amount of total sennosides of at least about 4.6% in weight, the extract free from resins, as well as the extract depleted in resins reconstituted with the resinous extract free from sennosides of the present invention, have merely traces of such active principles, as comprising an amount of total sennosides of at least ≤ 0.5% in weight.
Evidently, such percentages are, in practice, traces of total sennosides that cannot be accountable for the mucoadhesive and barrier properties exhibited by the extract of the invention, and that cannot induce the pharmacological activities for which senna or senna extracts are commonly used.
Since in one embodiment the extract is parallelly depleted also in resins, the latter being them also almost completely eliminated in the complete extraction process as described above, the mucoadhesive effect and the barrier effect observed for the extract depleted in resins according to the invention are not ascribable to resins either.
The extract of the invention can therefore be

a. resinous extract obtained in step b. of the process,
b. extract depleted in resins and sennosides, obtained in step d. of the process
c. mixture of the above-described extracts a. and b. in any desired ratio, e.g. 50% and 50% or other ratios, for instance by admixing the total amounts of both extracts obtained from the same initial material.

Each of such extracts is therefore useful for its mucoadhesion and barrier effect properties in all those cases in which the protection of skin or mucous membranes is needed or desirable; those may be cases in which a drug that attacks mucous membranes or skin has to be administered, therefore in order to prevent the damaging action of the drug, or in those cases in which the protection of an even partially compromised skin or mucous membrane is preferable or desirable, so as to enable a better and quicker healing thereof, defending it from further aggressions, or in those cases in which an individual has a chronic disorder in which skin or mucous membranes sustain irritations or alterations, therefore a barrier effect can prevent or limit damages on the skin or the mucous membrane.
In outlining the protection effect of mucous membranes, in the present description it is meant that such mucous membranes can be the oral mucosa, gastric mucosa, intestinal mucosa, the nasal mucosa, the uterine mucosa, the

rectal mucosa.

The present invention also provides a process for the preparation of the extract as described above, said process comprising the following steps:

a. preparing a hydroalcoholic extract of Senna leaves and/or pods (follicles) wherein said hydroalcoholic extract is prepared by subjecting *Cassia acutifolia* and/or *Cassia angustifolia* leaves and/or pods, optionally fragmented, to two or more extraction steps in ethanol at decreasing concentration

b. obtaining from the extract prepared in a. a concentrated aqueous extract and a resinous extract free from sennosides

c. concentrating said alcoholic extract by decantation and/or centrifugation and/or filtration, collecting the supernatant or the filtrate

d. said supernatant or filtrate in c. is subjected to one or more ultrafiltration steps whereby the permeate is collected and said permeate comprises total sennosides at a concentration in weight percentage ≤50.5% and wherein said one or more ultrafiltration steps is carried out on a semi-permeable membrane with a cutoff of about 500-15,000 daltons and/or

said filtrate or supernatant obtained in step c. or said permeate obtained after ultrafiltration is subjected to one or more steps through a high porosity adsorbing resin and the eluate is collected

wherein said eluate thus obtained comprises total sennosides at a concentration in weight percentage ≤0.5.

The extract obtained in step d. according to the method described above will have a concentration in weight percentage of total sennosides ≤0.5%.

The resinous extract obtained in step b. according to the method described above will be free from sennosides, which remain in the aqueous extract obtained always in b.

As already mentioned, object of the invention are both extracts, as well as a mixture thereof.

The process described herein could be carried out starting from senna leaves and/or pods (follicles, or fruits).

Alcoholic senna extracts could be treated according to the process reported from step b.

Dry senna extract could be treated according to the process reported from step a.

According to the invention, the above-described process can be carried out by performing at point a. at least two, at least three, or at least four steps in decreasing ethanol concentrations, in which the decreasing ethanol concentrations ranges from about 96% ethanol to about 0% ethanol.

For instance, step a. of the process may be carried out by performing a step in about 85% ethanol, a step in about 50% ethanol, and a step in about 20% ethanol.

Or, step a. of the process could be carried out by performing a step in about 85% ethanol, a step in about 50% ethanol, a step in about 20% ethanol and a step in about 5% ethanol.

The extracts obtained as described above are gathered to form a hydroalcoholic extract. These can be gathered, for instance, in equal mutual ratios, as 50:50 in the case of two steps in ethanol, or as 33.3:33.3:33.3 in the case of three steps in ethanol or 25:25:25:25 in the case of four steps in ethanol. Evidently, the extracts obtained by performing the various above-described steps in ethanol could be gathered in different ratios, according to the general knowledge of the technician in the field.

As already indicated above, the hydroalcoholic extract obtained at step a. will be used to prepare a concentrated aqueous extract at step b.

Said concentrated aqueous extract could be prepared by concentrating the hydroalcoholic extract obtained in a. by alcohol evaporation and it is decanted and/or centrifuged and/or filtered and the aqueous concentrated phase is collected (for instance, in the case of decantation and/or centrifugation) the aqueous supernatant is collected, whereas in the case of filtration the filtrate is collected. Alternatively, alcohol evaporation could be performed after the centrifugation and/or decantation and/or filtration stage.

Decantation could be performed, e.g., 1 to 72 hours, and could be followed or replaced by one or more centrifugations to about 3000-4000 (e.g., about 3500) rpm for a period comprised between 1 and 10 minutes, e.g. about 5 minutes. For filtration, panel filters with a cutoff from 10 to 50 micrometers could be used. The supernatant obtained after this step(s) is then collected and subjected, in case not already done before, to alcohol evaporation by standard techniques, like, e.g., by use of a thin film distillation system, or a batch concentration system according to standard protocols commonly used by the technician in the field.

The result of this evaporation is a concentrated aqueous extract that, in step c. of the process, is subjected to decantation and/or filtered.

The precipitate obtained after these steps is a resinous extract free from sennosides, it also object of the invention.

Therefore, object of the invention is also the process comprising only steps a. and b., in which the resinous extract obtained by sedimentation or precipitation is retained , i.e. a method for the preparation of a senna extract free

from sennosides, comprising the following steps:

a. preparing a hydroalcoholic extract of Senna leaves and/or pods (follicles)
b. obtaining from the extract prepared in a. an aqueous extract and a resinous extract free from sennosides, said resinous extract is collected.

In this case, of course, it is not necessary to concentrate the aqueous extract.
In the method comprising steps a-d, the concentrated aqueous extract (which could also be referred to as concentrated aqueous solution) may be subjected to decantation for a period comprised between 1 and 72 hours, and the supernatant is then recovered, thereby obtaining a clarified solution. Decantation may be followed or replaced by one or more centrifugations at about 3000-4000 (e.g., about 3500) rpm for a period comprised between 1 and 10 minutes, e.g. about 5 minutes.
Alternatively, the concentrated aqueous extract is filtered, thereby obtaining a clarified solution. Filtration could be performed, e.g., on a panel filter with a cutoff from 10 to 50 micrometers, like, e.g. a cutoff of 15-20 micrometers, or a cutoff of 25 micrometers, or even higher ones.
In a further embodiment the concentrated aqueous extract could be decanted as described above and the supernatant obtained could then be filtered as described above.
The supernatant or the filtrate obtained as described above are recovered and subjected to a step d. of depletion in total sennosides.
Furthermore, the method comprises a step d. of treatment of the supernatant or of the filtrate obtained in the above-described step c. before collection of the desired extract.
This step (d) could be carried out by ultrafiltration of the supernatant or filtrate obtained in c. and/or by passage through a high porosity adsorbing resin of the supernatant or filtrate obtained in c. or of the permeate obtained after ultrafiltration.
In the first case, the supernatant or filtrate obtained in c. is subjected to one or more ultrafiltration steps and the permeate is recovered, wherein said permeate comprises total sennosides at a concentration in weight percentage ≤0.5%.
In this further step, the aqueous extract (result of the filtration or supernatant of the decantation in c.) is subjected to one or more steps of on semi-permeable membranes with a molecular weight cutoff (NMCWO) of from 500 to 50,000 daltons, e.g. of 500, 1,000, 2,500, 5,000, 10,000, 20,000, 50,000 daltons. The permeate extract thus obtained is substantially free from total sennosides of resins and substances of terpenoid structure, whereas it is enriched in substances having a protective action and can be used as is or subjected to a lyophilization process to provide a lyophilized extract useful for formulations having therapeutic employ for internal and/or external use.
According to the invention, therefore, said one or more steps of ultrafiltration could be performed on membranes having a cutoff of about 500-50,000 daltons, e.g. about 10,000 daltons, 5,000 daltons and 2,500 daltons (corrected).
The present invention therefore also relates to a process for the preparation of a Senna extract comprising the following steps:

a. preparing a hydroalcoholic extract of Senna leaves and/or pods (follicles)
b. obtaining from the extract prepared in a. a concentrated aqueous extract and a resinous extract free from sennosides
c. decanting and/or filtering said aqueous extract collecting the supernatant or the filtrate
d. the supernatant or the filtrate obtained in c. is subjected to one or more ultrafiltration steps and the end permeate is recovered (collected),

wherein said permeate comprises total sennosides at a concentration in weight not greater than 0.5% (≤0.5%).
As already mentioned, optionally the extract obtained in b. and the extract obtained in d. may be admixed.
According to a further embodiment, the method of the invention could comprise, alternatively or additionally to step d. as described above, a step d'. wherein the filtrate or the supernatant obtained in c. or the permeate obtained after ultrafiltration is subjected to one or more steps through a high porosity adsorbing resin and the eluate, consisting of the substances not adsorbed to the resin, is collected, wherein said eluate thus obtained comprises total sennosides at a concentration in weight percentage ≤0.5%.
The present invention therefore also relates to a process for the preparation of a Senna extract comprising the following steps:

a. preparing a hydroalcoholic extract of Senna leaves and/or pods (follicles)
b. obtaining from the extract prepared in a. a concentrated aqueous extract and a resinous extract free from sennosides

c. decanting and/or filtering said aqueous extract collecting the supernatant or the filtrate
d'. the filtrate or the supernatant obtained in c., or the permeate obtained from one or more ultrafiltration steps as described above, is additionally subjected to one or more steps (passages) through a high porosity adsorbing resin.

wherein said eluate thus obtained comprises total sennosides at a concentration in weight percentage ≤0.5.

As already mentioned, optionally the extract obtained in b. and the extract obtained in d. may be admixed.

According to the present invention, the step in d' can be carried out on a column or with resins able to adsorb aromatic or highly apolar substances and to let elute non-aromatic polar substances. The resin-adsorbed material, if desired, could be desorbed with a suitable solvent, like e.g. ethanol or hydroalcoholic solvents, for other uses.

Suitable chromatography resins may be, e.g., high-porosity adsorption resins of styrene-divinyl benzene copolymer, like, e.g., amberlite XAD-2, serdolit PAD-II, ADS TQ 318.

In particular, the resin will be a resin able to adsorb aromatic and/or apolar substances, like, e.g., a hydrophobic adsorbing resin; the resin consists of microspheres of a diameter of 0.2 mm - 0.8 mm, with an uniformity coefficient ≤ 1.5 obtained by polymerization of Styrene and DVB without active groups, characterized by a highly porous physical structure and having the parameter relative to the pore volume equal to about 1.3 ml/g, enabling adsorption and selective elution of organic substances, preferably of aromatic nature. Therefore, the passage through resins having adsorbing features of the above-described type enables to obtain a fraction (extract of the invention) depleted in total sennosides (≤0.5%).

The extract obtained with the above-indicated processes will contain only traces also of other substances of terpenoid structure present in the initial product, besides the total sennosides.

The extract obtainable with the method of the invention comprises total sennosides at a concentration in weight not greater than 0.5% (≤0.5%, where this product is referred to the dry product).

The extracts according to the present invention can be used as-is or can be processed, e.g. by subjecting them to drying by lyophilization.

The extract obtained at d or d' has the features like, e.g., those reported in Table 1, where it is compared with the initial product, ultrafiltration permeate or resin eluate obtained in d and d', respectively.

Table 1

| Fraction | Yield | total sennosides (SFM) % |
|---|---|---|
| Initial product | | 4-6% |
| **Extract object of the invention (<10.000 daltons permeate obtained in step d) or eluate obtained in step d'** | 55% | ≤0.5% |
| **(resinous) extract object of the invention** | 10% | ≤0.05% |

The process according to the present invention, wherein the order of the steps as described above enables therefore to obtain a Senna extract depleted in total sennosides and surprisingly rich in substances having a protective action. The above-described process a. to d. also eliminates resins via the reagents and steps used, as evident to the technician in the field.

In one embodiment of the invention, the extract of the invention will also be substantially free from resins that will be found in the resinous extract obtained in b. and in the fractions discarded in c. and d or d'.

However, the resinous extract free from sennosides is collected and may be used as is or reunited with the extract obtained in d. or d'.

The present invention also relates to a composition comprising the extract in any one of the above-described embodiments useful in the protection of the skin or mucous membranes.

Said protection, of course with reference both to the extract per se and to the composition, could be a preventive protection or a protection of partially compromised skin or mucous membranes.

For instance, the composition or the extract can, by virtue of their barrier effect, facilitate the repair of damaged skin with entailed restoration of healthy and elastic skin.

The skin lesions according to the present invention are, e.g. lesions that may involve also tissue underlying the skin and in which no open wounds are present.

By skin lesions not implying the presence of open wounds, according to the present description, are meant those lesions in which the superficial layer of the skin, and the underlying layers, though not being wounded, are particularly fragile, irritated and damaged.

Non-limiting examples of this type of lesions are represented by first-degree burns, first-degree decubitus lesions, pressure lesions, newly cicatrized rashes, wounds or burns, irritations, erythemas.

The composition or the extract of the invention could then be used for the treatment or the prevention of skin lesions not involving the presence of open wounds, or in the prevention or slowing down of worsenings of the same.

The compositions according to the invention could advantageously comprise further components, e.g. of plant origin, having, e.g. emollient, digestive, pro-kinetic, cholagogic, carminative, prebiotic, relaxing, excipient, preserving, wetting properties.

When the composition or the extract of the invention are used for the protection of the skin, the application thereof will be topical. The embodiments of the compositions for topical use are reported hereinafter in the description.

The composition according to the invention could be made, e.g., as oil-in-water emulsion, water-in-oil emulsion, oil-in-gel or gel-in-oil emulsion, multiple emulsions, sprays, and anhydrous formulations (salve, gel, paste, cream, ointment) and will comprise one or more excipients suitable for the making of the desired end form.

Such excipients could be, e.g., emulsifying agents (cetearyl alcohol, cetearyl glucoside, hydrogenated castor oil) rheological additives, antioxidants (like, e.g., vitamins, tocopherols or other antioxidants known in the field).

The emulsifying agent could be a surfactant, which by lowering the interfacial tension decreases the free energy of the system; alternatively, also non-surfactant substances can be used, such as gum arabic, gelatin, hydrophilic colloids or finely subdivided powders (e.g., talc). In one embodiment, the excipients could be present at an overall concentration in weight comprised between 3 and 8%, such concentration being of course indicative, taking into account that anyhow the technician in the field will know how to adapt the concentration of the necessary excipients according to the embodiment he/she intends to prepare without addition of inventive activity.

Moreover, the composition could comprise perfuming and/or coloring agents which give it a pleasant smell, like, e.g., one or more essential oils like, e.g., lavender essential oil, melaleuca essential oil, lemon, mint, orange, and/or coloring agents which allow, e.g., to easily recognize the areas of application of the composition, the coloring being, e.g., temporary, so that it does not interfere with other later applications of the composition.

In one embodiment, said coloring and/or perfuming agents are comprised at an overall concentration in weight from 0.001 to 3%.

By "overall concentration in weight" it is meant the concentration in weight in the composition of the sum of the various excipients, or the concentration in weight in the composition of the sum of the various perfuming and/or coloring agents present in the composition.

As to the use of the composition in the protection of the skin, the invention also relates to medical devices like, e.g., medicated patches, medicated gauzes, medicated bandages, medicated towels, medicated pads, medicated diapers, i.e. patches, gauzes, bandages, towels, pads or diapers which comprise, or be at least partially covered by, or be at least partially imbued with the described composition of the invention in the most appropriate form.

The gauzes could be made as greasy gauzes, and so the bandages and the patches, using the composition made in the form, e.g., of an ointment, paste or cream. The towels could be imbued with the composition in the form of an oil emulsion with water or gel, and the diapers or the absorbent pads could be made by inserting the composition in the relevant layers known in the art for the insertion of protective or anti-irritating compositions.

Such products, like, e.g., infant diapers, absorbent pads (commonly called "sanitary pads") for women and adult incontinence pads, are commonly used, e.g., in infancy-, women- and geriatrics-related fields, and the technician in the field will know where to insert the composition described herein and which embodiment be the most suitable one without the need of particular teachings and only based on conventional techniques in the art.

Such devices will be applicable to the part to be treated and/or protected for a preventive purpose.

As already indicated above, the composition of the invention may be a composition for topical use which could be realized in the form of oil-in-water emulsion, water-in-oil emulsion, multiple emulsions, sprays, and anhydrous formulations (salve, ointment, gel, paste, cream, spray,) according to techniques commonly used in the field. The formulation indicated herein as "spray" could be an anhydrous formulation or even a formulation in emulsion, the form with an atomizer enabling also self-applications in zones harder to reach (like, e.g., the back), useful in all those cases in which the formulation is used, e.g., to alleviate sun rashes, erythemas, or skin irritations of various kind.

According to another embodiment, the extract or the composition of the invention could be used, thanks to their mucoadhesive and barrier effects, for the protection of the mucous membranes.

In this case as well, said protection could be a preventive protection, e.g. in all those cases envisaging an administration of drugs having the side effect of attacking the mucous membranes, or in those patients exhibiting conditions of recurring irritation of the same. In other cases, the protection could instead be a protection for curative purposes or in order to avoid the worsening of irritated or partially compromised mucous membranes.

In these cases, the administration of the extract or of the composition could be topical for all those mucous

membranes on which a topical administration is possible (e.g., oral, rectal, vaginal, nasal mucosa) or oral in the cases in which said membranes be, e.g., intestinal or gastric mucosal membranes.

The compositions for the protection of the mucous membranes could therefore be made in the form of capsule, tablet, lozenge, granule, powder, syrup, elixir, hard gelatine, soft gelatine, suspension, emulsion, solution, suppository, cream, gel, spray, ointment, pomade, paste, oil-in-water emulsion, water-in-oil emulsion, oil-in-gel emulsion, gel-in-oil emulsion.

In case of formulations for topical use, the above indications related to the compositions for the protection of the skin could be followed, or syrups, rinses, sprays could be made, e.g., for the protection of the mucous membranes of the mouth, throat, nose.

For application on the rectal mucosa, suppositories or enemas or microenemas could be used, with the excipients known to the technician in the field for the making of such compositions.

As already mentioned, the composition of the invention could be in the form of capsule, tablet, lozenge, hard gelatine, soft gelatine, granule, powder, syrup, elixir, suspension, emulsion. For oral administration the composition could be made in the form of daily unit dosages or of fractions of daily unit dosages (e.g. 2, 3, 4, 5, 6, or more capsules, tablets, lozenges, granule or powder single-doses, or gelatins could be taken over the day, in accordance with the judgment of the treating doctor), and may contain conventional excipients including, e.g., binding agents, like gum arabic, gelatine, sorbitol, gum tragacanth, and/or polyvinylpyrrolidone; fillers, like lactose, sugar, corn starch, rice starch, calcium phosphate, sorbitol and/or glycine; tableting lubricants, like magnesium stearate, talc, polyethylenglycol and/or silica; disintegrants, like potato starch; and moisturizers like sodium laurylsulphate. The tablets can be coated according to methods well-known in the standard pharmaceutical practice.

The composition could also be made in a liquid or semiliquid form, as a suspension, emulsion, solution for oral administration and could optionally contain natural aromatizing agents giving a palatable taste thereto.

The composition in the form of powder or granule could be pre-metered in suitable containers and ready for use, either by ingestion as such or to be resuspended in an appropriate liquid such as water, tea, etc. In this case as well, the composition could contain natural aromatizing agents giving a palatable taste thereto.

Evidently, all of the above-indicated excipients could be used in a pharmaceutically acceptable grade.

In one embodiment, the composition as described herein, in any one of the above-indicated embodiments, could be

in the form of pharmaceutical composition, i.e. comprise pharmaceutical-grade ingredients, or it could be or be introduced into a special-purpose food (medical food) or into a medical device.

The composition according to the present description could be made in the form of pharmaceutical composition or of medical device according to any one of the classes described in Directive 93/42/EEC on medical devices (comprising also substances and not only "devices" in the mechanical sense of the term), or in the form of medical food, food supplement, or in any form according to the regulatory provisions of the Country in which said composition will be produced.

The medical food could also contain as ingredients other components comprising, e.g., combinations of vitamins, mineral salts and other substances aimed at diet supplementing.

The extract or the composition of the invention is therefore useful for its mucoadhesion and barrier effect properties in all those cases in which the protection of skin or mucous membranes is needed or desirable; those may be cases in which a drug that attacks mucous membranes or skin has to be administered, therefore in order to prevent or limit the damaging action of the drug, or in those cases in which the protection of a partially compromised skin or mucous membrane is preferable or desirable so as to enable a better and quicker healing thereof, defending it from further aggressions, or in those cases in which an individual has a chronic disorder in which skin or mucous membranes sustain irritations or alterations, therefore a barrier effect can prevent or limit damages on the skin or the mucous membrane.

The invention also relates to a method for the treatment or for the prevention the onset or the worsening of skin lesions not involving the presence of open wounds, wherein such method comprises one or more applications of the composition of the invention or of the medical device comprising it once or more per day on the concerned part.

The application of the composition, for instance, could be repeated whenever needed (e.g. at each change of pad in case of prevention of incontinence-related rashes) or once, twice, thrice, four or more times per day in general.

The invention also relates to a method for the preparation of a composition according to any one of the embodiments described above, wherein a Senna extract depleted in total sennosides, wherein said total sennosides have a concentration in weight percentage $\leq$0.5%, is mixed with at least one of an excipient and/or a substance of natural and/or plant origin having emollient, digestive, pro-kinetic, cholagogic, carminative, prebiotic, relaxing, excipient, preserving, wetting properties as described above.

Among these, e.g., could be used one or more of: gentian root extract, boldo leaf extract, milk thistle fruit extract, artichoke leaves extract, dandelion root extract, anise fruit extract, rosmary leaf extract, mint leaf extract, marjoram leaf extract, cumin fruit extract, coriander fruit extract, ginger root extract, fennel fruit extract, caraway fruit extract, charcoal,

inulin, aloe leaf gel.

Object of the present invention is also a method for use in the protective (preventive or curative) treatment of the skin or the mucous membranes providing the administration of the extract or the composition of the present invention to a patient in need thereof. Such administration could also be concomitantly with the administration of other drugs.

The absence of pharmacologically active principles of senna in the extract or in the composition makes such products particularly suitable to administration concomitantly with other drugs, as a side effect of interaction between the extract or the composition of the invention and the drug coadministered or concomitantly administered is quite unlikely.

A non-limiting example of the method of treatment and/or of prevention of the skin or the mucous membranes, could comprise the administration, subdivided into a single dose or plural doses, described of the mixture or composition according to the present description, for a period of time of between one and six weeks, e.g. of between three and six weeks or even for a period of time higher than six weeks, in accordance with the judgment of the treating doctor.

Such administration could precede the administration of the drug even for a prolonged period, so as to optimize the health state of the skin or of the mucous membrane to be treated.

The treating doctor will know how to establish both the most appropriate dosage and the administration times also on the basis of the patient's health state, weight, sex and age.

One of the substantial differences between the structure of the skin and that of the mucous membranes is represented by the absence, in the mucous membranes, of a selective barrier such as the corneous layer. Oral mucous membranes contact with noxious or irritating substances present in the environment (pollutants, pathogenic microorganisms, etc.) can therefore cause a high penetration of said substances both inside the mucous membranes and in the related airways (bronchi, lungs, etc.) causing inflammatory and/or allergic pathologies.

The protective effect of the extract of the present invention was assessed by mucoadhesion assays and barrier effect assays.

The former aims at evaluating the mucoadhesivity of the product under examination in order to establish whether such product has the ability to adhere to the mucous membranes and consequently exert a protective action.

For instance, two simple models are available for evaluating mucoadhesivity of the product of interest. The first one, e.g., evaluates the mucoadhesive ability of the sample by measuring its ability to bond to mucin disposed on an inclined plane, allowing the moving away by sliding of the sample without mucoadhesive ability. The second one enables to evaluate the mucoadhesive ability of the sample by inhibition of the lectin/glycoprotein bond expressed on the cell membrane, of cells coming from a mucosal membrane.

For evaluation of the barrier effect an *in vitro* model is available enabling to evaluate the impediment of the sample to transit of LPS, accountable, in the model, for the freeing of mediators such as interleukin 6 (IL-6), measured semi-quantitatively by ELISA assay.

**Evaluation of the percentage of inhibition of the lectin/glycoprotein bond**

In the first model, mucoadhesivity was determined by evaluation of the percentage of inhibition of the lectin/glycoprotein bond. In this model, for instance, buccal or vaginal mucosal cells may be used. The cells were initially treated with biotinylated lectin (Con-A), having high affinity for the glucoside and mannoside residues present in the glycoproteins of the membrane. The sites of the glycoproteins of the mucosal membranes will thus be occupied with the biotinylated lectin. The presence of the biotin (vitamin H) in the lectin is indispensable for the next stage. The cells already treated with biotinylated lectin are charged with streptavidin peroxidase, making it possible to form the protein/glucose/lectin/biotin/streptavidin peroxidase complex. At this point, the cells are washed and the protein/glucose/lectin/biotin/streptavidin peroxidase complex is quantified, thanks to the presence of the peroxidase, by means of a reaction of oxidation of the ortho-phenylenediamine.

The protein/glucose/lectin/biotin/streptavidin peroxidase complex catalyses the polymerization reaction:

$$\text{O-phenylenediamine} \xrightarrow[\text{Strep.perox}]{H_2O_2} \text{2,3-diaminophenazine (yellow)}$$

The intensity of the yellow/orange coloration of the solution (measured using a spectrophotometer with $\lambda$=450 nm) is proportional to the quantity of glycoprotein/lectin bonds and therefore to the quantity of available sites (glycoproteins) for mucoadhesion. The absorbency value thus determined constitutes the "control".

In the mucoadhesion assay reported below, the mucosal cells are treated for about 15 minutes at 30°C with the product under examination before the treatment with lectin. In the presence of mucoadhesive products, said products will inhibit lectin bonding, decreasing, proportionally to their mucoadhesion ability, the signal strength in the sample compared to the control as described above.

The percentage of mucoadhesion of the product (%MA) could be determined as

$$\%MA = (1 - \text{abs sample}/\text{abs control}) \times 100$$

**Evaluation of adhesion percentage by inclined plane with mucin.**

Measurements are performed by an equipment comprised of a 45°-inclined plane, thermostated to 37°C; below said plane a microbalance is set, which performs measurements at regular 1-s intervals and prints recorded measurements on paper. Performed measurements are then transcribed and reprocessed on Excel spreadsheet.

The inclined plane acts as support for the biological substrate, consisting of a mucin film set up by depositing, on a plexiglas plane kept horizontal, a volume equal to 3 ml of a suspension of porcine gastric mucin at an 8% concentration (w/w) in a phosphate buffer, pH 6.4.

The dispersion is then brought to dryness at the temperature of 44°C so as to obtain a film of known surface area equal to 33.6 $cm^2$.

An exactly weighted amount of the extract according to the invention is dissolved in a solvent. The extract is then deposited on the measurement plane with a multichannel pipette at constant time and rate during 6 seconds.

The multichannel pipette is calibrated so as to meter the solution (or dispersion) over the work plane. The total amount is measured at each assay (the weight of said volume is measured before the assay and depends on the density of the sample under analysis).

The sample, which will fall on the microbalance at the end of the run, is loaded in the top part of the inclined plane, containing the mucin film, and let slide thereon. The amount of fallen sample is measured by recording the weight variation as a function of time. The assay as made herein lasts 40 seconds, though usually after 20 seconds there is no weighing variation.

Measurements in the absence of the mucin film on the inclined plane are also performed (measurements indicated as blank) with the same amounts of sample and under the same assaying conditions.

Thus, it is possible to assess the intrinsic sliding properties of the sample, regardless of its ability to interact with the biological substrate.

The adhered % is calculated: defined as the amount of sample remained adhered to the mucin film or to the inclined plane (without mucin, blank) at the end of the measurement, normalized with respect to the amount deposited on the inclined plane.

Moreover, it is determined the percentage difference between sample amount adhered to the plane with mucin and sample amount adhered to the plane without mucin. The calculation is performed in accordance with the following equation:

$$\%\ \text{difference} = (\text{adhered mucin}\ \% - \text{adhered blank}\ \%)/\text{adhered}\ \%\ \text{of the blank}$$

where

- adhered mucin % = sample percentage remained adhered to the mucin film at the end of the measurement
- adhered blank % = sample percentage at the inclined plane at the end of the blank measurements.

Barrier effect assay

The barrier effect assay is a biological-type *in vitro* assay employed for assessing the ability of finished products and/or raw materials to protect, through the formation of a thin "insulating" layer, mucous membranes and skin from contact with environmental contaminants (dust, pollens, microorganisms, etc.).

The assay was developed to simulate *in vitro* the action exerted by products that are applied on skin and/or mucous membranes with the aim of creating a protective film against external aggressors.

The model takes advantage of the principle whereby cells subjected to contact with an inflammatory agent produce and secrete pro-inflammatory mediators (cytokines) in the extracellular environment in an amount related to the degree of inflammation caused; within a certain range, a direct proportionality exists between concentration and times of exposure to the inflammatory agent and amounts of released cytokines.

The experimental model adopted provides two chambers physically separated by a semi-permeable membrane (0.4μm pores). Cells are seeded in the lower chamber, whereas the upper chamber accommodates the inflammatory agent; on the semi-permeable membrane separating the two chambers a thin film of the sample under analysis is stratified to highlight, if present, a barrier effect to the free transit of the inflammatory agent.

The semi-permeable membrane allows passage of the inflammatory agent in the lower chamber and constitutes the support on which the sample to be assayed is stratified. Depending on the "insulating" ability of the sample, a decrease of LPS migration from the upper chamber to the bottom one will be had (therefore a lesser stimulation of cells to cytokine production).

EXAMPLES

**Example 1**

**Preparation of extract**

Senna leaves and/or pods (follicles) were fragmented and subjected to four steps of extraction in ethanol at decreasing concentrations; performing a step in 85% ethanol, a step in 50% ethanol, a step in 20% ethanol, a step in 5% ethanol.

The alcoholic extracts obtained as described were gathered in a 25:25:25:25 ratio and subjected to centrifugation for a time of 1-5 minutes at a rotation rate equal to 3500 rpm; the supernatant was recovered, and concentrated by ethanol evaporation with use of a thin film distillation system according to the standard protocol, providing the feeding of the extract to be concentrated at a rate of about 500 l/h; operation was by setting a residual vacuum of 0.6-0.8 bars and the fluid heating the evaporation walls was set at 140°C, thereby obtaining, after ethanol elimination, a concentrated aqueous extract.

The precipitate, or the resinous extract, was collected and proved to be free from sennosides.

The aqueous extract thus obtained was filtered on a panel filter with a cutoff of 25 micrometers.

The filtrate thus obtained, which comprised total sennosides at a concentration in weight not greater than al 5% was subjected to a step of ultrafiltration on membrane with a cutoff of 10,000 daltons and/or 5,000 daltons and/or 2,500 daltons according to standard methodologies.

The end permeate thus obtained comprised total sennosides at a concentration in weight not greater than 0.5%.

The concentrated aqueous extract, after supernatant settling and recovery or after permeate filtration and recovery, was subjected, alternatively or additionally to the above-described ultrafiltration process, to a treatment with high-porosity adsorption resin; in particular, a resin of styrene-DVB copolymer of ADS TQ 318 type was used. Evidently, any adsorbing resin with similar features could be used in the realization of the present invention.

In this case a standard process of obtainment of the extract by treatment on resin entails the introduction of the concentrated aqueous extract into a chromatographic column containing the adsorption resin. The feeding fluid must have suspended solids indicatively comprised between 0.2 to 10° Bix, The feed rate is comprised between 1 and 4 BV/hour, and preferably 2BV/hour. The corresponding aqueous eluate is collected and subjected to drying by lyophilization, or to desiccation, The resin-adsorbed substances are eluted by using 96% ethyl alcohol, or methanol or acetone, or aqueous mixtures with the solvents reported hereto, preferably 96% ethyl alcohol. In this latter case, the alcoholic eluate is subjected to desiccation or to lyophilization (after having added, in this latter case, water in a 1:1 v/v ratio with the alcoholic eluate and having evaporated the ethanol), useful for other purposes.

The remaining sennosides are retained by the resin and the eluate is collected. By this process, it is obtained an extract comprising total sennosides at a concentration not greater than 0.5% (titer referred to the dried product, e.g. by lyophilization) and, when both steps d. (ultrafiltration and adsorption on resin) are performed, sennosides concentration is further lowered.

The following examples report the data on the extract depleted in resins and sennosides and on the resinous extract depleted in sennosides according to the invention.

**Example 2**

**Assessment of the mucoadhesive ability of the extract of the invention by evaluation of the percentage of inhibition of the lectin/glycoprotein bond**

The oral cavity of 8-10 male and female donors (age: 20-35 years), without food for at least 60 minutes, was gently scraped with a wooden spatula and the cells immersed in 0.05 M TBS (Tris Buffer Saline) pH 7,6. After count with 0.5% Tripan blue, the cells were diluted with 0.9% NaCl until obtaining a final concentration of 480,000 cells for each sample to be used in the assaying. Cells were kept at a temperature of 4°C. Cellular suspensions were then centrifuged at 2000 rpm for 5 minutes and incubated with 5 ml of a solution comprising 0.5% extract of the invention. For the control, the cellular suspensions were instead incubated with 5 ml of 0.9% NaCl. Incubation was carried on for 15 minutes at the temperature of 30°C, with gentle stirring. After 3 washings with TBS, the buccal cells were incubated with 5 ml of 10 mg L-1 of Con-A at 30°C for 30 minutes, washed 3 times with TBS and then incubated at 30°C for 60

minutes with 5 ml of 5 mg L-1 of streptavidin peroxidase. After 3 washings with TBS, 240,000 cells per sample were added to 2.5 ml of o-phenylendiamine (o-pd) in 0.05M phosphate citrate and $H_2O_2$ and the reaction was stopped after 5 minutes with 1 M $H_2SO_4$.

Then, absorbency values for the individual determinations were read by spectrophotometer (experiment carried out in triplicate). Since the composition has a coloration that might interfere with the exact determination of the absorbency value recorded at the end of the protocol, for sample reading a (0.9%) NaCl solution (for the control) or the solution of the product suitably diluted with the 0.9% NaCl solution were used as blank. The individual samples were assayed 3 times and the results reported represent the averages ± SEM of all assays performed.

| **Sample** | **% mucoadhesion** SENNA EXTRACT OBTAINED IN D | **% mucoadhesion SENNA EXTRACT RESINS** |
|---|---|---|
| A | 48 | 31.5 |
| B | 50 | 34 |
| C | 53 | 28.9 |
| Average ± S.D. | 50± 2.50 | 31.5± 2.55 |

The results obtained demonstrate that the assayed extract possesses a high mucoadhesive ability with respect to oral cavity mucosal membranes. In light of the results obtained, it is possible to state that the composition, demonstrating to possess high, resistant mucoadhesivity, can play an interesting protective role on mucosal cells..

**Example 3**

**Assessment of mucoadhesive ability of the extract of the invention on an inclined plane**

Measurements were performed by means of an equipment consisting of a 45°-inclined plane, thermostated to 37°C; below said plane a microbalance was set, which performed measurements at regular 1-s intervals and printed recorded measurements on paper. Performed measurements were transcribed and reprocessed on Excel spreadsheet.

The inclined plane acts as support for the biological substrate, consisting of a mucin film. The mucin film was set up by depositing, on the plane consisting of plexiglas kept horizontal, a volume equal to 3ml of a suspension of porcine gastric mucin at an 8% concentration (w/w) in a phosphate buffer, pH 6.4.

The dispersion was brought to dryness at the temperature of 44°C so as to obtain a film of known surface area equal to 33.6 $cm^2$. Amount, concentration and deposition plane were defined during process development.

An exactly weighted amount of the extract of the invention was dissolved in solvent (ethanol 50°, subsequently diluted 1:2 in MEM Eagle-type medium with Earle's BSS; sample concentration: 10 mg/ml or 1%). The extract was deposited on the measurement plane with a multichannel pipette at constant time and rate during 6 seconds.

The multichannel pipette was adjusted so as to meter 250 microliters of solution (or dispersion) over the work plane. The total amount measured at each assay is of about 1 ml per sample (the weight of said volume is measured before the assay and depends on the density of the sample under analysis).

The sample, which would fall on the microbalance, was loaded in the top part of the inclined plane, containing the mucin film, and let slide thereon.

The amount of fallen sample was measured by recording the weight variation as a function of time.

The assay lasted 40 seconds, however it is stressed that after 20 seconds there was no weighing variation.

Measurements in the absence of the mucin film were also performed (measurements indicated as blank) with the same amounts of sample and under the same assaying conditions, but without the mucin layer deposited on the plane.

Thus, it is possible to assess the intrinsic sliding properties of the sample, regardless of its ability to interact with the biological substrate.

The adhered % is calculated: defined as the amount of sample remained adhered to the mucin film or to the inclined plane (without mucin, blank) at the end of the measurement, normalized with respect to the amount deposited on the inclined plane.

Moreover, it is determined the percentage difference between sample amount adhered to the plane with mucin and sample amount adhered to the plane without mucin. The calculation is performed in accordance with the following equation:

% difference = (adhered mucin % - adhered blank %)/adhered % of the blank.

where

- adhered mucin %= sample percentage remained adhered to the mucin film at the end of the measurement
- adhered blank % = sample percentage at the inclined plane at the end of the blank measurements.

Analysed samples:

The measurements were performed for the following samples:

- 50% EtOH in water: used as negative control, i.e. as a solution without mucoadhesion
- 0.7% sodium alginate in water used as positive control, i.e. as a substance with known mucoadhesive properties
- Senna sample: lyophilized Senna fractions, 1% solution in 50% EtOH.

**Results**

In the graphs reported in Figure 3 (panels A, B, C, D) are represented, respectively, the sliding profiles obtained with the negative control (50% EtOH in water), the positive control (0.7% sodium alginate), the sample under analysis (soluble Senna fraction in 50% EtOH, 1% solution) and the sample under analysis (non-soluble Senna fraction in 50% EtOH, 1% solution).

From the figures, it is evident how the slid amount of mucoadhesive substances in the presence of mucin be at each time definitely lower than that fallen in blank measurements, to indicate occurred interaction between the formulation and the biological substrate. Said difference remains at the end of the measurement.

The figures also demonstrate that, despite the elimination of resins (eliminated along with total sennosides), the Senna extract according to the present invention keeps a good mucoadhesive ability.

In the table below, mucoadhesion parameters are reported, measured as described in the experimental section.

The percentage of sample adhered to the inclined plane without mucin and with mucin was assessed, and the percentage difference between the two values was calculated, for the extract obtained with the process according to claim 5, steps a-d, for sodium alginate, which is a substance with known mucoadhesive ability, and for the solvent.

Table 2 A

| | % DIFFERENCE (between % adhered to the inclined plane with mucin and without mucin) |
|---|---|
| Extract of the invention (% of sample adhered to mucin film - % of sample adhered to the plane without mucin)/ % of sample adhered to the plane without mucin | **117%** |
| 0.7% ALGINATE SODIUM, (% of sample adhered to the mucin film- % of sample adhered to the plane without mucin)/ % of sample adhered to the plane without mucin | **127%** |
| 50% Ethanol (% of sample adhered to the mucin film- % of sample adhered to the plane without mucin)/ % of sample adhered to the plane without mucin | **23%** |

Table 2 B

| | % DIFFERENCE (between % adhered to the inclined plane with mucin and without mucin) |
|---|---|
| Extract of the invention - RESINS (% of sample adhered to the mucin film- % of sample adhered to the plane without mucin)/ % of sample adhered to the plane without mucin | **25%** |

(continued)

| | % DIFFERENCE (between % adhered to the inclined plane with mucin and without mucin) |
|---|---|
| 0.7% ALGINATE SODIUM, (% of sample adhered to the mucin film- % of sample adhered to the plane without mucin)/ % of sample adhered to the plane without mucin | **127%** |
| 50% Ethanol (% of sample adhered to the mucin film- % of sample adhered to the plane without mucin)/ % of sample adhered to the plane without mucin | **23%** |

In this case as well, the results evidently show how the extract object of the invention has mucoadhesive and sliding properties similar to those of a known mucoadhesive substance like sodium alginate.

## Example 4.

### Barrier effect assay

For the barrier effect assay, two chambers physically separated by a semi-permeable membrane (0.4μm pores) were used. Human fibroblast cells were seeded in the lower chamber, whereas the inflammatory agent LPS (purified E. Coli lipopolysaccharide) was introduced into the upper chamber; on the semi-permeable membrane separating the two chambers a thin film of senna extract was stratified as described in the present invention.

Induced inflammatory response was estimated through semi-quantitative dosage of Interleukin-6 (IL-6) released in the culture medium of the lower chamber: Barrier effect assessment was obtained by comparison with the positive control in which the two chambers were separated by the same type of semi-permeable membrane, free however from any barrier.

As to the threshold value above which it may be said that a substance causes a barrier effect in the assay reported herein, a value equal to 15% of inhibition as compared to the control was identified by the Inventors, during the setting up of the assay, on the basis of assays performed on substances known to have a barrier effect.

Then, the barrier effect of the extract as described herein was evaluated.

The data reported below show how the extract has a remarkable barrier effect.

The assessment assay was carried out as follows through a method developed to simulate *in vitro* the protective action of substances and formulations which, when applied to the skin and mucous membranes in vivo, form an "insulating" effect against environmental agents.

The model takes advantage of the principle whereby cells subjected to contact with an inflammatory agent produce and secrete pro-inflammatory mediators (cytokines) in the extracellular environment in an amount related to the degree of inflammation caused. The greater the amount of inflammatory agent reaching the cells, the greater the amount of cytokines released.

The model envisages the arrangement of two chambers physically separated by a semi-permeable membrane which allows the passage of sufficiently small-sized solutes.

In the lower chamber, consisting of a well containing plates for cell cultures, HuDe cells (no. BS PRC 41) are grown, while the upper chamber, consisting of an insert for complex cell cultures (transwells), accommodates the inflammatory agent.

On the surface of the semi-permeable membrane of the insert separating the two chambers, before the introduction of the inflammatory agent in the upper chamber, a thin film of the sample being examined is stratified to assess any BE upon the free passage of the inflammatory agent.

As a function of the insulating capabilities of the sample, there will be had a decrease of the migration of the inflammatory agent from the upper chamber and, as a consequence, a lesser stimulation of the cells to produce cytokines. The extent of the inflammatory reaction is estimated through the semi-quantitative dosage of cytokines released in the culture medium of the lower chamber, in particular of interleukin 6 (IL-6).

As a control a similar experiment is used in which no sample is stratified on the membrane, thus making it possible to measure the effect of the inflammatory agent without any barrier in addition to the semi-permeable membrane.

Further, an internal control is used in which the cultured cells are pre-treated with the substance adapted to induce the release of the marker and the sample is placed on the semi-permeable membrane in the absence of said substance, one or more measurements in time are thus performed of the quantity of marker in the culture medium of said internal control. In the internal control, the cells are therefore stimulated first with the inducing substance and then

there has to be assessed whether the sample which may pass the membrane and go into the cells pushed by the medium above has any effect in decreasing the release of the marker not related to the barrier effect. For instance, when an inflammatory agent is used as the inducing substance, the internal control makes it possible to understand if the reduction in the concentration of cytokines in the culture medium is due to the barrier effect or if the sample that may pass into the cells pushed by the medium above has any effect in reducing the inflammatory response independently of the barrier effect.

The barrier effect (BE) is expressed as a percentage of the reduction of the release of IL-6 and is calculated through comparison with the positive control in which the two chambers are separated by the same type of semi-permeable membrane without the barrier created by the sample.

4.1 PREPARATION OF THE CELL CULTURE:

For each assayed sample, cells of the HuDe cell line were seeded in the wells of a cell culture plate, one for the barrier assay (BA) and another one for the internal control at a density of 40,000 cells /ml in MEM medium supplemented with 10% bovine serum (FBS); 1 ml of cell suspension per well.

The cells are treated with the SAMPLE (CAM), with the POSITIVE CONTROL (C+) (inflammatory agent without sample), and with the NEGATIVE CONTROL (C) (medium only) and each assay is carried out in triplicate.

The plates were incubated at 37°C, overnight (22-24 hours).

4.2 PREPARATION OF INSERTS FOR COMPLEX CELL CULTURES

Inserts for complex cell cultures (Becton Dickinson) are placed on other plates, and on each of them a fixed amount of collagen of 0.1 ml/ml is supplied. The plates were incubated at 37 °C overnight (22-24 hours).

**DAY 2**

4.3 VERIFICATION OF STATE AND LEVEL OF CELL CONFLUENCY

In order to proceed with the experiment, a confluency not lower than 95% is required.

4.4 COLLAGEN LAYER DRYING

From the two plates (BA and IC) with the inserts the collagen is removed and the insert left under the flow of the hood for the time required to let them dry completely (10÷15 minutes)

**4.5 BARRIER ASSAY (BA)**

The steps described below are carried out in the culture plate for the BA

Setting up of the sample layer in the BA:

On the sample's semi-permeable membrane 100μl of a 0.5% alginate-based composition were inoculated and allowed to stratify for 20 minutes while in the C+ and C - inserts nothing is added. Once the 20 minutes have passed the excess sample is eliminated and the membranes are washed with PBS according to procedures specified by the protocol

Addition of LPS (inflammatory agent) to BA inserts

Once the sample layer has dried, in the first three CAM inserts and in the three C+ ones, 300μl of the LPS (membrane lipopolysaccharide) were inoculated at the concentration of 1μg/ml while in the remaining three of the C- 300 μl of MEM medium with 5% FBS were added. The inserts are inserted in their respective wells with the cells and the plates are incubated for 1 hour at 37°C and under an atmosphere enriched with 5% $CO_2$ overnight.

Once the 1 h incubation is completed, the inserts are removed and discarded and the plates are incubated again overnight (22-24 hours).

**4.6 INTERNAL CONTROL (IC) ASSAY:**

The internal control assay was carried out concomitantly with the BT

Exposure of IC cells to LPS:

Once dried, in the first six inserts of IC, three for the sample to be analysed and three for the C+, 300μl of the LPS solution are inoculated while in the remaining three of the C- 300μl of the medium are added
The inserts with LPS and MEM are then inserted in the wells with IC cells and all incubated for 1 h.

LPS removal and IC membrane drying:

Once the 1 h incubation is completed, the inserts are removed from the wells with the cells and transferred to the empty plate while the plate with the cells is placed in an incubator.
The LPS solution still present is removed from the inserts, the latter are subjected to a rapid wash with ultrapure sterile water and allowed to dry.

Arrangement of sample layer in the IC:

On the semi-permeable membrane of the three inserts for the sample 100μl of a 0.5% alginate-based composition were inoculated and allowed to stratify for 20 minutes while in the C+ and C - inserts nothing is added. Once the 20 minutes have passed the excess sample is eliminated and the membranes are washed with PBS according to procedures specified by the protocol.

Addition of LPS to IC inserts

Once the inserts with the sample are ready, 300 μl of medium are added to all inserts (CAM, C+, C-). The inserts are inserted in their respective wells with the cells and the plates are incubated for 1 h at 37°C.
Once the 1 h incubation is completed, the inserts are removed and discarded and the plates incubated again overnight (22-24 hours).

**DAY 3**

4.7 SUPERNATANT COLLECTION AND ENZYME IMMUNOASSAY

Once the 22-24 hours have passed, the supernatants are collected from the BA and the IC plates for performing the ELISA assay and semi-quantitative dosage of IL-6.

BARRIER EFFECT (BE) ASSESSMENT

The BE of a substance or compound is expressed as *% reduction in the release of IL-6 cytokine* by cells exposed to LPS wherein the sample has been assayed relative to the positive control (C+) wherein the cells have only been exposed to LPS.

**BE = % reduction in release of IL-6 cytokine = 100 - [(pg/μL cytokines released from sample/pg/μL cytokines released from C+) x 100]**

Table 1, reported below, shows that the senna extract obtained according to the present invention creates a barrier to passage of LPS.

| SAMPLE | % INHIBITION OF IL-6 |
|---|---|
| Internal control | 0 |
| SENNA, EXTRACT DEPLETED IN RESINS AND SENNOSIDES ACCORDING TO THE PRESENT INVENTION | 47 |
| SENNA, RESINOUS EXTRACT DEPLETED IN SENNOSIDES ACCORDING TO THE PRESENT INVENTION | 60 |

As may be seen from the above results, the extract depleted in sennosides and resins has excellent mucoadhesive properties and a high barrier effect; the resinous extract depleted in sennosides, through having surprisingly reduced mucoadhesive properties, has a very high barrier effect.

Hence, evidently both extracts are suitable for the uses described and claims, as well as mixtures of the two make it possible to increase the barrier effect keeping high mucoadhesive properties.

## Claims

1. A *Cassia acutifolia* and/or *Cassia angustifolia* extract depleted in total sennosides, wherein said total sennosides have a concentration in weight percentage ≤0.5%.

2. The extract according to claim 1 further depleted in resins.

3. The extract according to claim 1 or 2 for use in the treatment or protection of partially compromised skin or mucous membranes.

4. The extract for use according to claim 3 wherein said mucous membrane is the oral mucosa, gastric mucosa, intestinal mucosa, the nasal mucosa, the vaginal mucosa, the uterine mucosa, the rectal mucosa.

5. A process for the preparation of a *Cassia acutifolia* and/or *Cassia angustifolia* extract comprising the following steps:

   a. preparing a hydroalcoholic extract of *Cassia acutifolia* and/or *Cassia angustifolia* leaves and/or pods wherein said hydroalcoholic extract is prepared by subjecting *Cassia acutifolia* and/or *Cassia angustifolia* leaves and/or pods, optionally fragmented, to two or more extraction steps in ethanol at decreasing concentration
   b. obtaining from the extract prepared in a. a clarified alcoholic extract and a resinous extract
   c. concentrating said alcoholic extract by decantation and/or centrifugation and/or filtration, collecting the supernatant or the filtrate
   d. said supernatant or filtrate in c. is subjected to one or more ultrafiltration steps whereby the permeate is collected and said permeate comprises total sennosides at a concentration in weight percentage ≤50.5% and wherein said one or more ultrafiltration steps is carried out on a semi-permeable membrane with a cutoff of about 500-15,000 daltons and/or

   said filtrate or supernatant obtained in step c. or said permeate obtained after ultrafiltration is subjected to one or more steps through a high porosity adsorbing resin and the eluate is collected
   wherein said eluate thus obtained comprises total sennosides at a concentration in weight percentage ≤0.5.

6. The process according to claim 5 wherein the resinous extract obtained in b. and the extract depleted of sennosides obtained in d. are admixed.

7. The process according to claim 5 or 6 wherein said decreasing ethanol concentration in step a. ranges from about 96% ethanol to about 0% ethanol.

8. The process according to any one of claims 5-7 wherein said two or more steps in a. comprise a step in about 85% ethanol, a step in about 50% ethanol, and a step in about 20% ethanol.

9. The process according to claim 8 further comprising a step in about 5% ethanol.

10. The process according to any one of claims 5 to 9 wherein said filtration in c. is carried out with a filter with a porosity of about 25 micrometers.

11. The process according to any one of claims 5 to 10 wherein said passage through resin is carried out on a styrene and DVB copolymer high porosity adsorbing resin.

12. A composition comprising the extract according to any one of claims 1 or 2.

13. The composition according to claim 12 for use in the treatment or protection of partially compromised skin or mucous membranes.

14. The composition for use according to claim 13 wherein said mucous membrane is the oral mucosa, gastric mucosa, intestinal mucosa, the nasal mucosa, the vaginal mucosa, the uterine mucosa, the rectal mucosa.

15. The composition for use according to any one of claims 13 to 14, further comprising substances of natural and/or plant origin having emollient, digestive, pro-kinetic, cholagogic, carminative, prebiotic, relaxing, excipient, preserving, wetting properties.

16. The composition for use according to any one of claims 13 to 15 in the form of capsule, tablet, lozenge, granule, powder, syrup, elixir, hard gelatine, soft gelatine, suspension, emulsion, solution, suppository, cream, gel, spray, ointment, pomade, paste, oil-in-water emulsion, water-in-oil emulsion, oil-in-gel emulsion, gel-in-oil emulsion.

17. The composition for use according to any one of claims 13 to 16 wherein said composition is a pharmaceutical composition, is comprised in a medical device, or is comprised in a medical food.

**Patentansprüche**

1. *Cassia acutifolia*- und/oder *Cassia angustifolia-Extrakt* mit abgereicherten Gesamtsennosiden, wobei die Gesamtsennoside über einen Gewichtsanteil von ≤ 0,5 % verfügen.

2. Extrakt gemäß Anspruch 1, der weiterhin über abgereicherte Harze verfügt.

3. Extrakt gemäß Anspruch 1 oder 2 zur Verwendung bei der Behandlung oder zum Schutz von zum Teil beeinträchtigter Haut oder Schleimhäuten.

4. Extrakt zur Verwendung gemäß Anspruch 3, wobei die Schleimhaut die Mundschleimhaut, Magenschleimhaut, Darmschleimhaut, die Nasenschleimhaut, die Vaginalschleimhaut, die Uterusschleimhaut, die Rektalschleimhaut ist.

5. Verfahren zur Herstellung eines *Cassia acutifolia*- und/oder *Cassia angustifolia*-Extrakts, das die folgenden Schritte umfasst:

   a. Zubereiten eines wässrig-alkoholischen Extrakts aus *Cassia acutifolia*- und/oder *Cassia angustifolia*-Blättern und/oder -Schoten, wobei der wässrigalkoholische Extrakt dadurch zubereitet wird, dass *Cassia acutifolia*- und/oder *Cassia angustifolia*-Blätter und/oder -Schoten, optional fragmentiert, einem oder mehreren Extraktionsschritten in Ethanol bei abnehmender Konzentration ausgesetzt werden.
   b. Erhalten, von dem in a. zubereiteten Extrakt, eines geklärten alkoholischen Extrakts und eines harzigen Extrakts.
   c. Konzentrieren des alkoholischen Extrakts durch Dekantieren und/oder Zentrifugieren und/oder Filtrieren, wobei der Überstand oder das Filtrat aufgefangen werden.
   d. Der Überstand oder das Filtrat in c. werden einem oder mehreren Ultrafiltrationsschritten ausgesetzt, wobei das Permeat aufgefangen wird und das Permeat umfasst: Gesamtsennoside mit einem Gewichtsanteil von ≤ 0,5 %, und wobei der eine oder die mehreren Ultrafiltrationsschritte auf einer semipermeablen Membran mit einer Trennung von ungefähr 500 - 15.000 Daltons ausgeführt werden und/oder

   das Filtrat oder der Überstand, die in Schritt c. erhalten werden, oder das Permeat, das nach einer Ultrafiltration erhalten wird, werden einem oder mehreren Schritten durch ein adsorbierendes Harz hoher Porosität ausgesetzt und das Eluat wird aufgefangen,
   wobei das Eluat, das so erhalten wird, Gesamtsennoside mit einem Gewichtsanteil von ≤ 0,5 umfasst.

6. Verfahren gemäß Anspruch 5, wobei der harzige Extrakt, der in b. erhalten wird, und der von Sennosiden abgereicherte Extrakt, der in d. erhalten wird, eingemischt werden.

7. Verfahren gemäß Anspruch 5 oder 6, wobei die abnehmende Alkoholkonzentration in Schritt a. von ungefähr 96 % Ethanol bis ungefähr 0 % Ethanol reicht.

8. Verfahren gemäß einem der Ansprüche 5 - 7, wobei die zwei oder mehr Schritte in a. umfassen: einen Schritt in ungefähr 85 % Ethanol, einen Schritt in ungefähr 50 % Ethanol und einen Schritt in ungefähr 20 % Ethanol.

9. Verfahren gemäß Anspruch 8, das weiterhin einen Schritt in ungefähr 5 % Ethanol umfasst.

10. Verfahren gemäß einem der Ansprüche 5 bis 9, wobei die Filtration in c. mit einem Filter mit einer Porosität von ungefähr 25 Mikrometern ausgeführt wird.

11. Verfahren gemäß einem der Ansprüche 5 bis 10, wobei die Passage durch Harz auf einem adsorbierenden Styrol- und DVB-Copolymer-Harz hoher Porosität ausgeführt wird.

12. Zusammensetzung, die ein Extrakt gemäß einem der Ansprüche 1 oder 2 umfasst.

13. Zusammensetzung gemäß Anspruch 12 zur Verwendung bei der Behandlung oder zum Schutz von zum Teil beeinträchtigter Haut oder Schleimhäuten.

14. Zusammensetzung zur Verwendung gemäß Anspruch 13, wobei die Schleimhaut die Mundschleimhaut, Magenschleimhaut, Darmschleimhaut, die Nasenschleimhaut, die Vaginalschleimhaut, die Uterusschleimhaut, die Rektalschleimhaut ist.

15. Zusammensetzung zur Verwendung gemäß einem der Ansprüche 13 bis 14, die weiterhin umfasst: Substanzen natürlichen und/oder pflanzlichen Ursprungs, die über weichmachende, verdauungsfördernde, prokinetische, durchfallerregende, karminative, präbiotische, entspannende, Hilfsstoff-, konservierende, befeuchtende Eigenschaften verfügen.

16. Zusammensetzung zur Verwendung gemäß einem der Ansprüche 13 bis 15 in der Form einer Kapsel, einer Tablette, einer Lutschtablette, eines Granulats, eines Pulvers, eines Sirups, eines Elixiers, einer Hartgelatine, einer Weichgelatine, einer Suspension, einer Emulsion, einer Lösung, eines Zäpfchens, eine Creme, eines Gels, eines Sprays, einer Salbe, einer Pomade, einer Paste, einer Öl-in-Wasser-Emulsion, einer Wasser-in-Öl-Emulsion, einer Gel-in-Öl-Emulsion.

17. Zusammensetzung zur Verwendung gemäß einem der Ansprüche 13 bis 16, wobei die Zusammensetzung eine pharmazeutische Zusammensetzung ist, in einem Medizinprodukt enthalten ist, oder in einer medizinischen Ernährung enthalten ist.

**Revendications**

1. Extrait de *Cassia acutifolia* et/ou de *Cassia angustifolia* appauvri en sennosides totaux, dans lequel lesdits sennosides totaux ont une concentration, en pourcentage en poids, $\leq$ 0,5 %.

2. Extrait selon la revendication 1, en outre appauvri en résines.

3. Extrait selon la revendication 1 ou 2, pour une utilisation dans le traitement ou la protection de membranes muqueuses ou cutanées partiellement compromises.

4. Extrait pour une utilisation selon la revendication 3, dans lequel ladite membrane muqueuse est la muqueuse orale, la muqueuse gastrique, la muqueuse intestinale, la muqueuse nasale, la muqueuse vaginale, la muqueuse utérine, la muqueuse rectale.

5. Procédé pour la préparation d'un extrait de *Cassia acutifolia* et/ou de *Cassia angustifolia,* comprenant les étapes suivantes :

a. préparation d'un extrait hydro-alcoolique de feuilles et/ou de cosses de *Cassia acutifolia* et/ou de *Cassia angustifolia,* dans lequel ledit extrait hydro-alcoolique est préparé par soumission de feuilles et/ou de cosses de *Cassia acutifolia* et/ou de *Cassia angustifolia,* éventuellement fragmentées, à deux ou plus de deux étapes d'extraction dans de l'éthanol à une concentration décroissante,
b. obtention, à partir de l'extrait préparé en a., d'un extrait alcoolique clarifié et d'un extrait résineux,
c. concentration dudit extrait alcoolique par décantation et/ou centrifugation et/ou filtration, collecte du surnageant ou du filtrat,
d. soumission dudit surnageant ou filtrat, obtenu en c., à une ou plusieurs étapes d'ultrafiltration, en conséquence

de quoi le perméat est collecté et ledit perméat comprend des sennosides totaux à une concentration, en pourcentage en poids, ≤ 0,5 % et dans lequel lesdites une ou plusieurs étapes d'ultrafiltration sont effectuées sur une membrane semi-perméable avec une séparation d'environ 50-15000 daltons, et/ou

soumission dudit filtrat ou surnageant obtenu en c., ou dudit perméat obtenu après ultrafiltration, à une ou plusieurs étapes sur une résine adsorbante fortement poreuse, et collecte de l'éluat,
dans lequel ledit éluat ainsi obtenu comprend des sennosides totaux à une concentration, en pourcentage en poids, ≤ 0,5.

**6.** Procédé selon la revendication 5, dans lequel l'extrait résineux obtenu en b., et l'extrait appauvri en sennosides obtenu en d., sont mélangés.

**7.** Procédé selon la revendication 5 ou 6, dans lequel ladite concentration décroissante d'éthanol dans l'étape a. va d'environ 96 % d'éthanol à environ 0 % d'éthanol.

**8.** Procédé selon l'une quelconque des revendications 5 à 7, dans lequel lesdites deux ou plus de deux étapes en a. comprennent une étape dans environ 85 % d'éthanol, une étape dans environ 50 % d'éthanol et une étape dans environ 20 % d'éthanol.

**9.** Procédé selon la revendication 8, comprenant en outre une étape dans environ 5 % d'éthanol.

**10.** Procédé selon l'une quelconque des revendications 5 à 9, dans lequel ladite filtration en c. est effectuée avec un filtre ayant une porosité d'environ 25 micromètres.

**11.** Procédé selon l'une quelconque des revendications 5 à 10, dans lequel ledit passage sur une résine est effectué sur une résine adsorbante fortement poreuse de copolymère de styrène et de DVB.

**12.** Composition comprenant l'extrait selon l'une quelconque des revendications 1 et 2.

**13.** Composition selon la revendication 12, pour une utilisation dans le traitement ou la protection de membranes muqueuses ou cutanées partiellement compromises.

**14.** Composition pour une utilisation selon la revendication 13, dans laquelle ladite membrane muqueuse est la muqueuse orale, la muqueuse gastrique, la muqueuse intestinale, la muqueuse nasale, la muqueuse vaginale, la muqueuse utérine, la muqueuse rectale.

**15.** Composition pour une utilisation selon l'une quelconque des revendications 13 à 14, comprenant en outre des substances d'origine naturelle et/ou végétale ayant des propriétés émollientes, digestives, prokinétiques, cholagogues, carminatives, prébiotiques, relaxantes, d'excipient, conservatrices, mouillantes.

**16.** Composition pour une utilisation selon l'une quelconque des revendications 13 à 15, sous la forme de capsule, comprimé, pastille, granule, poudre, sirop, élixir, gélule en gélatine dure, gélule en gélatine molle, suspension, émulsion, solution, suppositoire, crème, gel, spray, onguent, pommade, pâte, émulsion huile dans l'eau, émulsion eau dans l'huile, émulsion huile dans gel, émulsion gel dans l'huile.

**17.** Composition pour une utilisation selon l'une quelconque des revendications 13 à 16, dans laquelle ladite composition est une composition pharmaceutique, est comprise dans un dispositif médical, ou est comprise dans un aliment thérapeutique.

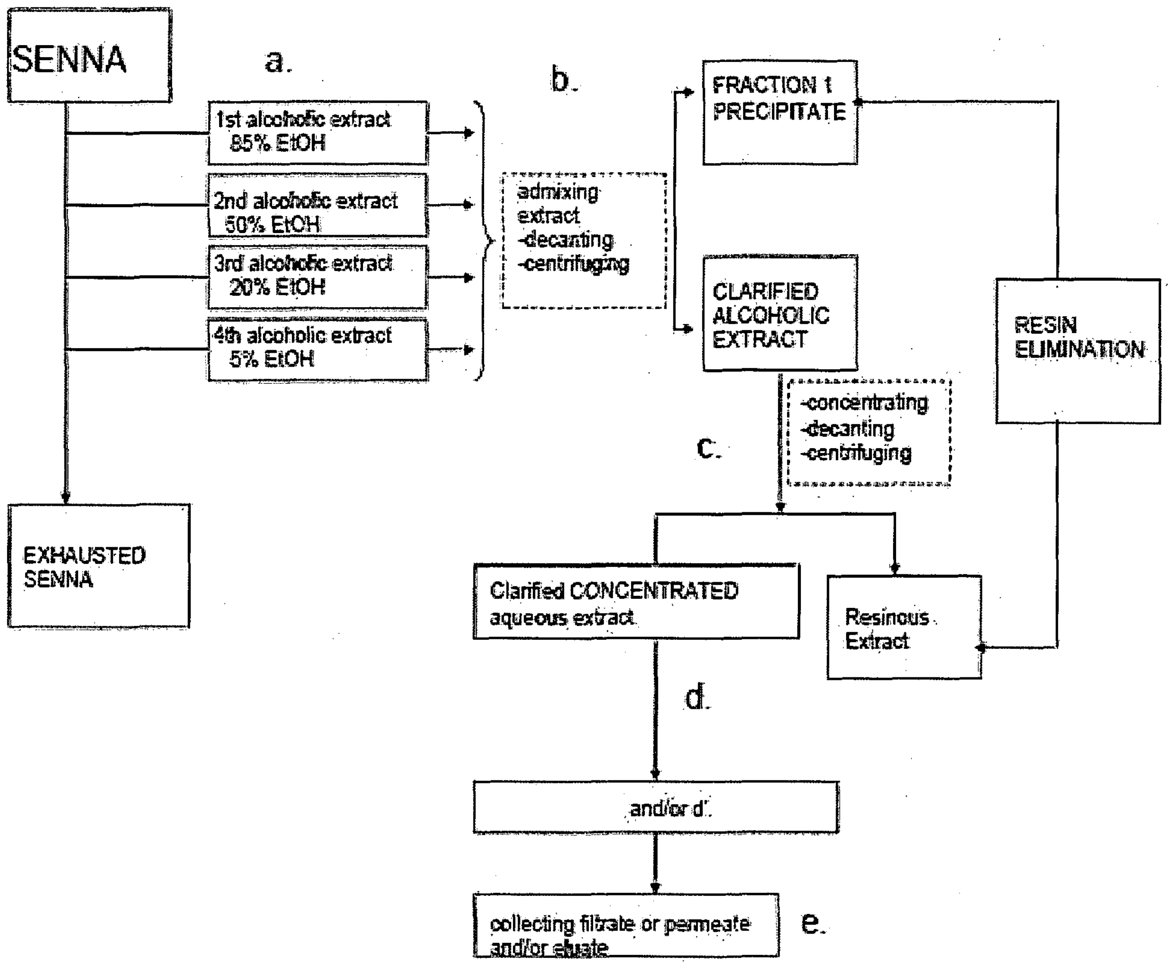
SENNA
a.
1st alcoholic extract 85% EtOH
2nd alcoholic extract 50% EtOH
3rd alcoholic extract 20% EtOH
4th alcoholic extract 5% EtOH
b.
admixing extract
-decanting
-centrifuging
FRACTION 1 PRECIPITATE
CLARIFIED ALCOHOLIC EXTRACT
RESIN ELIMINATION
-concentrating
-decanting
-centrifuging
c.
EXHAUSTED SENNA
Clarified CONCENTRATED aqueous extract
Resinous Extract
d.
and/or d'.
collecting filtrate or permeate and/or eluate
e.

Fig. 1

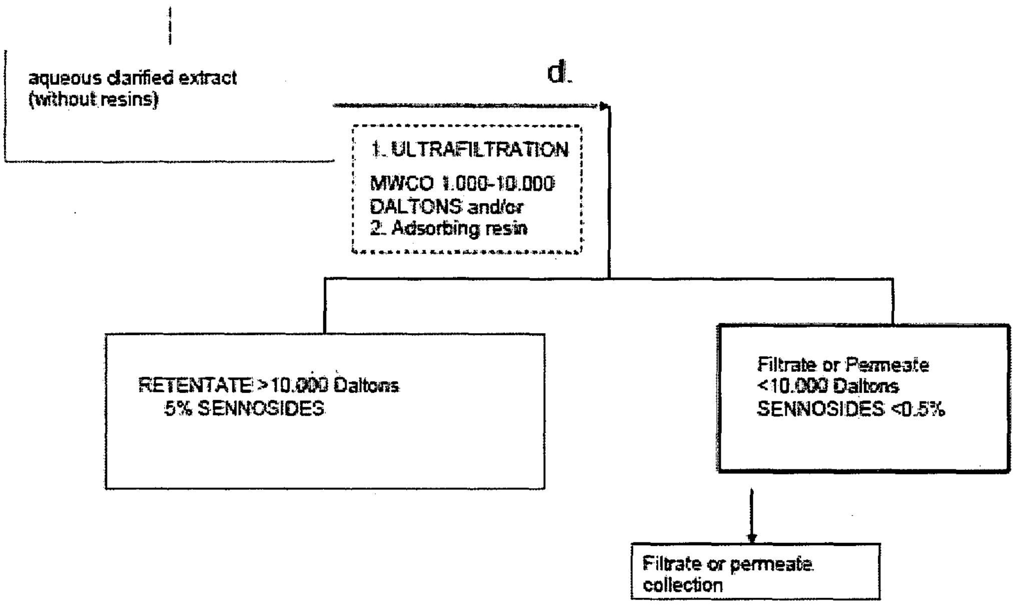
aqueous clarified extract
(without resins)
d.
1. ULTRAFILTRATION
MWCO 1.000-10.000
DALTONS and/or
2. Adsorbing resin
RETENTATE >10.000 Daltons
5% SENNOSIDES
Filtrate or Permeate
<10.000 Daltons
SENNOSIDES <0.5%
Filtrate or permeate
collection

Fig. 2A

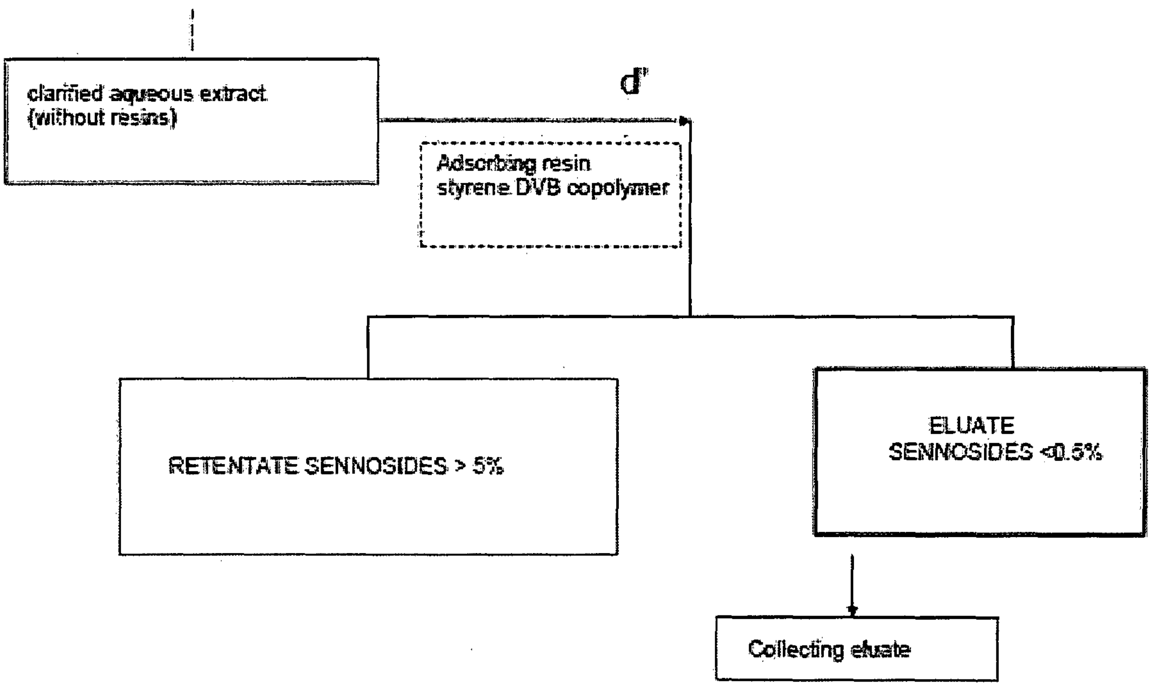
clarified aqueous extract
(without resins)
d'
Adsorbing resin
styrene DVB copolymer
RETENTATE SENNOSIDES > 5%
ELUATE
SENNOSIDES <0.5%
Collecting eluate

Fig. 2B

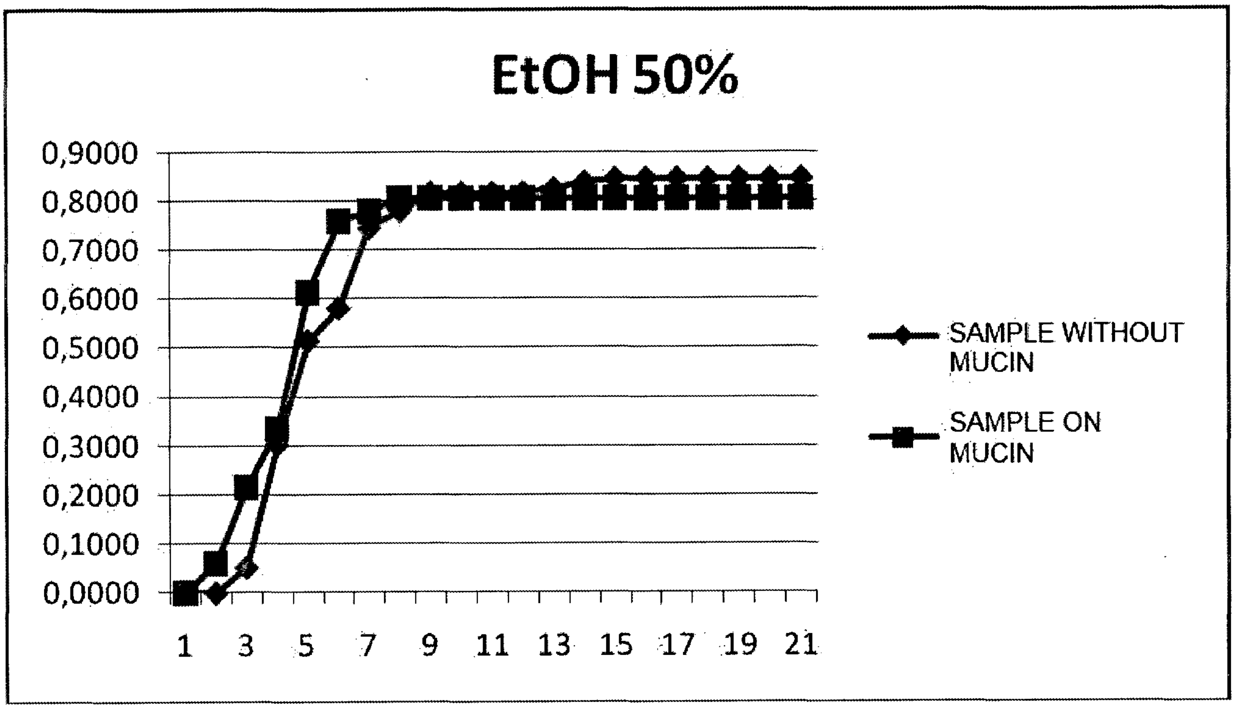
EtOH 50%
0,9000
0,8000
0,7000
0,6000
0,5000
0,4000
0,3000
0,2000
0,1000
0,0000
1 3 5 7 9 11 13 15 17 19 21
SAMPLE WITHOUT MUCIN
SAMPLE ON MUCIN

Fig. 3A

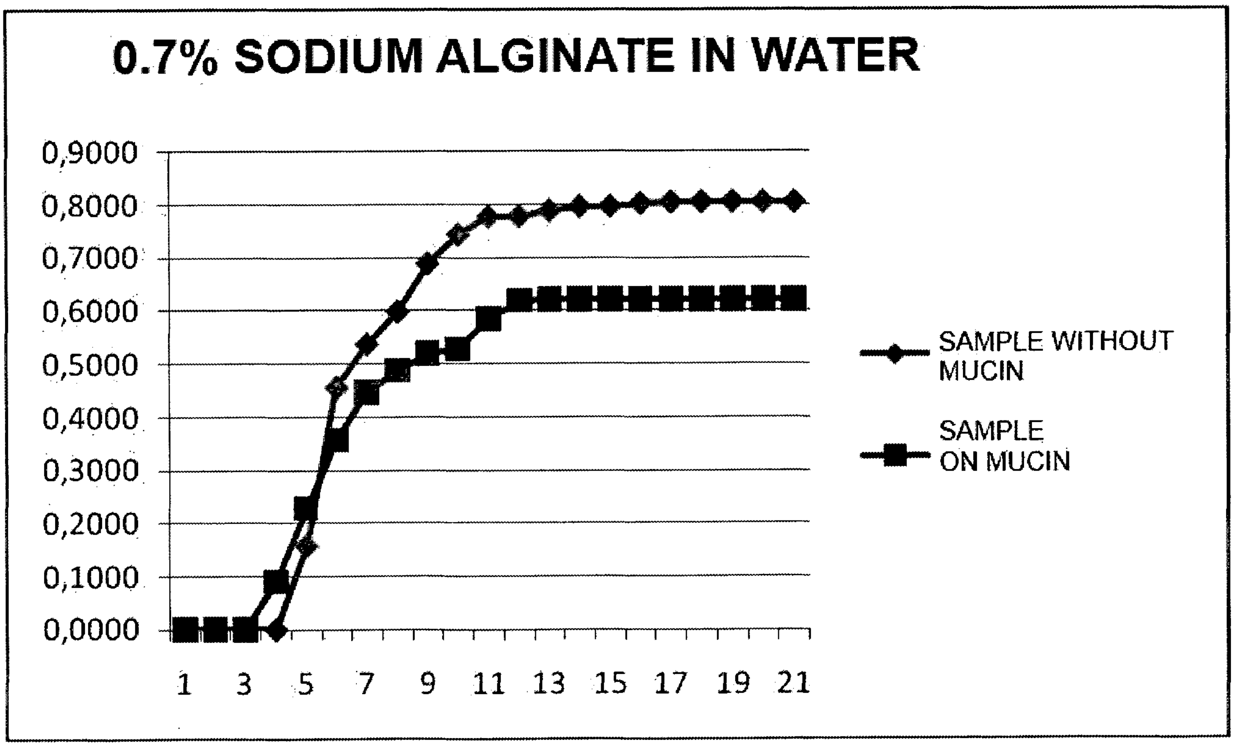
0.7% SODIUM ALGINATE IN WATER
0,9000
0,8000
0,7000
0,6000
0,5000
0,4000
0,3000
0,2000
0,1000
0,0000
1 3 5 7 9 11 13 15 17 19 21
SAMPLE WITHOUT MUCIN
SAMPLE ON MUCIN

Fig 3B

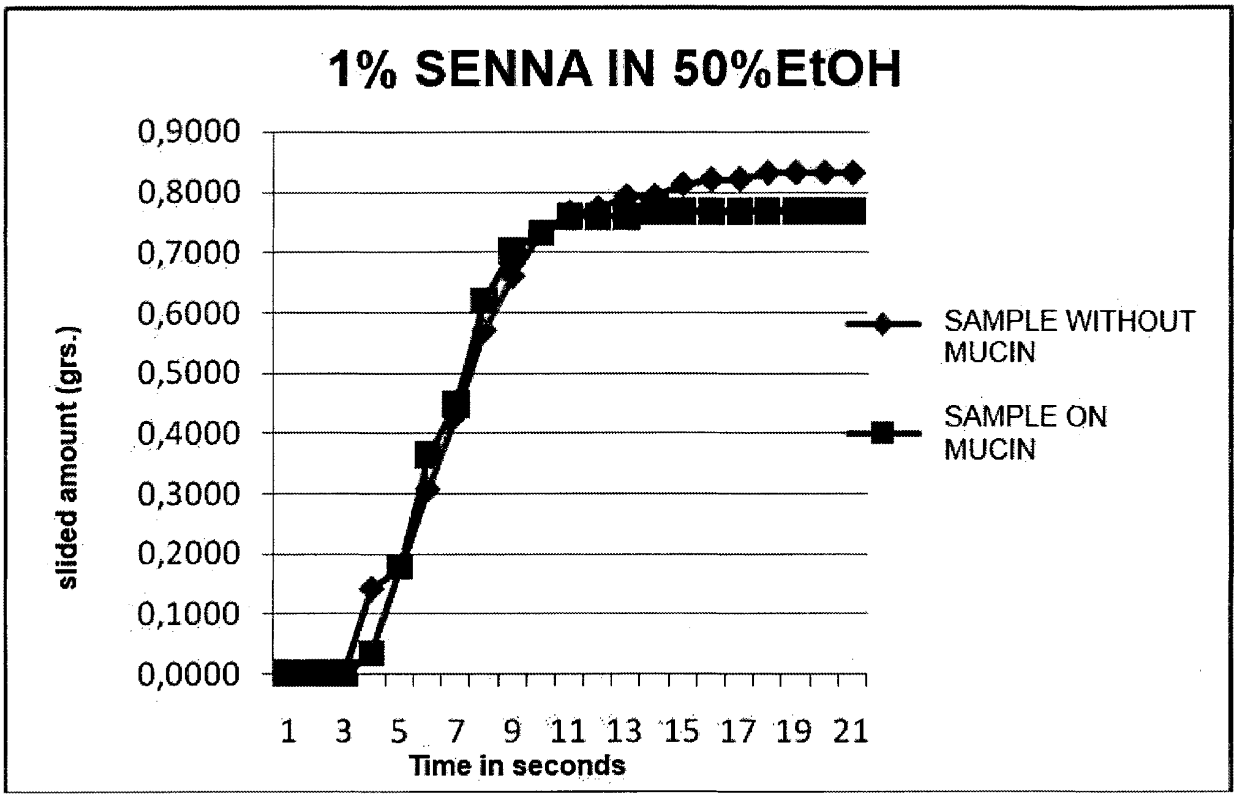
1% SENNA IN 50%EtOH
0,9000
0,8000
0,7000
0,6000
0,5000
0,4000
0,3000
0,2000
0,1000
0,0000
slided amount (grs.)
1 3 5 7 9 11 13 15 17 19 21
Time in seconds
SAMPLE WITHOUT MUCIN
SAMPLE ON MUCIN

Fig 3C

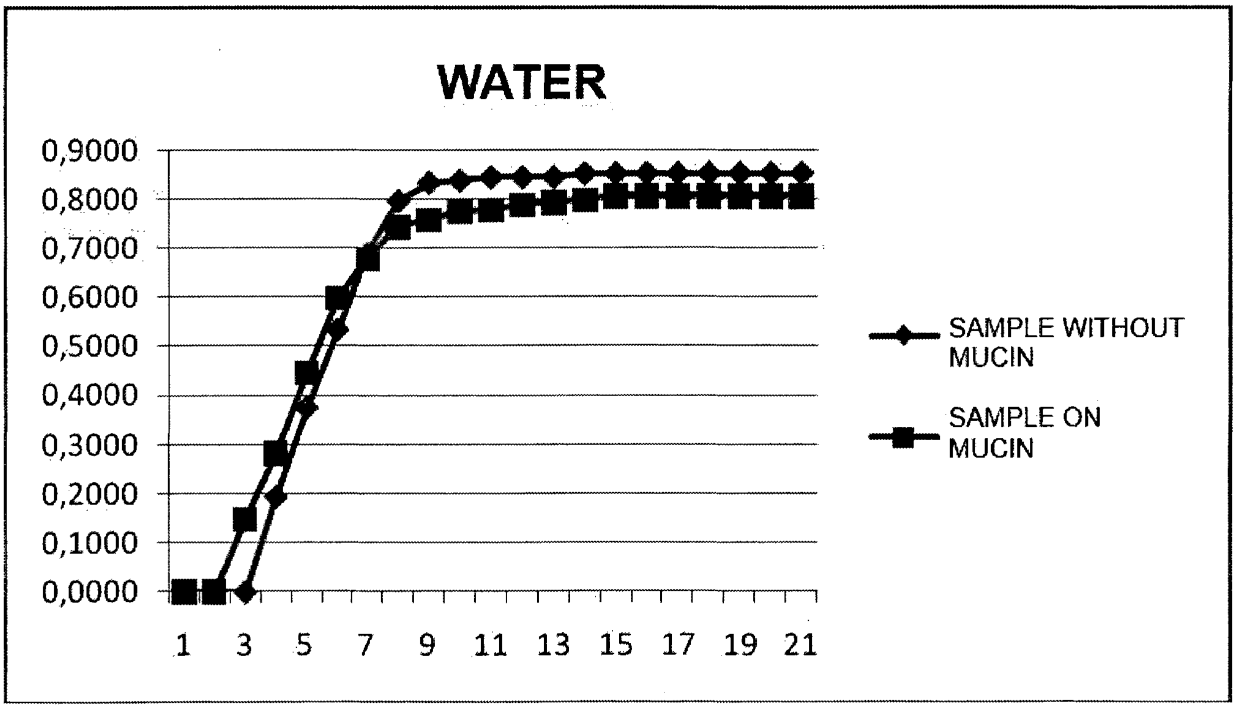
WATER
0,9000
0,8000
0,7000
0,6000
0,5000
0,4000
0,3000
0,2000
0,1000
0,0000
1 3 5 7 9 11 13 15 17 19 21
SAMPLE WITHOUT MUCIN
SAMPLE ON MUCIN

Fig 3D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- Sennae folium. European Pharmacopoeia 7.0. 2011, 1236, , 1237